# FASCICULE DE BREVET EUROPEEN

(11) **EP 0 900 078 B1**
(45) Date de publication et mention de la délivrance du brevet: **02.02.2000**
(21) Numéro de dépôt: 97919465.1
(22) Date de dépôt: 02.04.1997
(51) Int. Cl.: A61K 31/19

(54) **UTILISATION DE DERIVES BENZONAPHTALENIQUES POUR LA FABRICATION DE MEDICAMENTS DESTINES AU TRAITEMENT DES MALADIES DU SYSTEME NERVEUX**
VERWENDUNG VON BENZONAPHTALENE-DERIVATEN ZUR HERSTELLUNG EINES ARZNEIMITTELS ZUR BEHANDLUNG VON KRANKHEITEN DES NERVENSYSTEMS
USE OF BENZONAPHTHALENE DERIVATIVES TO MAKE MEDICAMENTS FOR TREATING DISEASES OF THE CENTRAL NERVOUS SYSTEM

(30) Priorité: 05.04.1996 FR 9604359
(43) Date de publication de la demande: 10.03.1999
(73) Titulaire: Galderma Research & Development, S.N.C., 06560 Valbonne (FR)
(72) Inventeur: VIGE, Xavier, F-91160 Longjumeau (FR); BENAVIDES, Jésus, F-92290 Châtenay-Malabry (FR); SHROOT, Braham, F-06600 Antibes (FR); TAUPIN, Véronique, F-75013 Paris (FR)
(74) Mandataire: Tezier Herman, Béatrice
(86) Numéro de dépôt international: FR9700590
(87) Numéro de publication internationale: WO9737648

(56) Documents cités:
- US-A- 5 455 248
- DATABASE MEDLINE US NATIONAL LIBRARY OF MEDICINE (NLM), BETHESDA, MD, US an: 96178020, SHALITA A. ET AL.: "a comparison of the efficacy and safety of adapalene gel 0.1% and tretinoin gel 0.0025% in the treatment of acne vulgaris: a multicenter trial" XP002035679 & J AM ACAD DERMATOL, vol. 34, no. 3, Mars 1996, pages 482-485,

## Description

La présente invention concerne l'utilisation des composés répondant à la formule (I) donnée ci-après et de leurs sels, pour la fabrication de médicaments destinés au traitement des neuropathies périphériques, des maladies neurodégénératives centrales et des maladies auto-immunes du système nerveux. 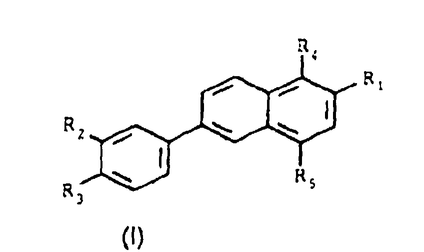 ces composés étant décrits, ainsi que leur procédé de préparation, dans le brevet EP 0 199 636.

Dans la formule (I) :
- R₁ représente un groupe -COR₆ ou CH₂OH, R₆ représentant un radical 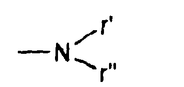 ou un radical OR₇, R₇ représentant un atome d'hydrogène, un radical alkyle ayant de 1 à 20 atomes de carbone, un radical monohydroxyalkyle, ou un radical polyhydroxyalkyle, r' et r" représentant un atome d'hydrogène, un radical alkyle inférieur, un radical mono ou polyhydroxyalkyle, un radical aryle éventuellement substitué ou un reste d'amino-acide ou de sucre aminé ou encore pris ensemble forment un hétérocycle,
- R₂ représente un atome d'hydrogène, un radical alkyle, ramifié ou non, ayant de 1 à 15 atomes de carbone, un radical alcoxy ayant de 1 à 4 atomes de carbone, ou un radical cycloaliphatique,
- R₃ représente un atome d'hydrogène, un radical hydroxyle, un radical alkyle, ramifié ou non, ayant de 1 à 4 atomes de carbone, un radical alcoxy ayant de 1 à 10 atomes de carbone,un radical cycloaliphatique substitué ou non, un radical thio-cycloaliphatique ou un radical de formule -O-Si(CH₃)₂-R₈, R₈ représentant un radical alkyle inférieur linéaire ou ramifié,
- R₄ et R₅, identiques ou différents, représentent un atome d'hydrogène,un radical alkyle inférieur, un radical hydroxyle ou un radical acyloxy inférieur.

Selon la présente invention, parmi les radicaux alkyle, ramifié ou non, ayant de 1 à 20 atomes de carbone ou de 1 à 15 atomes de carbone, on peut citer avantageusement les radicaux méthyle, éthyle, isopropyle, butyle, tertiobutyle, hexyle, octyle, nonyle, 2-éthyl-hexyl et dodécyle. De préférence, ces radicaux présentent de 1 à 12 atomes de carbone.

Lorsqu'il est inférieur, le radical alkyle comprend généralement de 1 à 6 atomes de carbone. On peut citer, comme radical alkyle inférieur, les radicaux méthyle, éthyle, propyle, isopropyle, butyle, tertiobutyle, hexyle.

Par radical monohydroxyalkyle ou polyhydroxyalkyle, on doit entendre un radical contenant de 1 à 6 atomes de carbone et de 1 à 5 groupes hydroxyles.

Parmi les radicaux monohydroxyalkyle, on préfère un radical contenant de préférence 1 ou 3 atomes de carbone, notamment les radicaux hydroxyméthyl, 2-hydroxyéthyl, 2 ou 3-hydroxypropyle.

Parmi les radicaux polyhydroxyalkyle, on préfère un radical présentant de 3 à 6 atomes de carbone et de 2 à 5 groupes hydroxyles, tels que les radicaux 2,3-dihydroxypropyle, 2,3,4-trihydroxybutyle, 2,3,4,5-tétrahydroxypentyle ou le reste du pentaérythritol.

Parmi les radicaux aryle, on préfère un radical phényle, thiophène ou pyridine, éventuellement substitué par au moins un atome d'halogène, un radical hydroxyle, un radical alkyle, une fonction nitro, un groupe méthoxy ou une fonction amine éventuellement, substituée. On préfère le radical phényle éventuellement substitué.

Par reste d'aminoacide, on entend notamment un reste dérivant de l'un des acides aminés tels que la lysine, la glycine ou l'acide aspartique, et par reste de peptide, on entend plus particulièrement un reste de dipeptide ou de tripeptide résultant de la combinaison d'acides aminés.

Parmi les restes de sucre aminé, on peut citer les restes dérivés de glucosamine, galactosamine et mannosamine.

Par hétérocycle, on entend de préférence un radical pipéridino, morpholino, pyrrolidino ou pipérazino, éventuellement substitué en position 4 par un radical alkyle en C₁-C₆ ou polyhydroxyalkyle tels que définis ci-dessus.

Parmi les radicaux alcoxy ayant de 1 à 10 atomes de carbone, on peut citer notamment le radical méthoxy, éthoxy, isopropoxy, hexyloxy et décyloxy.

Parmi les radicaux alcyloxy inférieur, on entend des radicaux présentant de 1 à 4 atomes de carbone, comme par exemple les radicaux acétyloxy ou propionyloxy.

Par radical cycloaliphatique, on entend un radical alcane cyclique ou polycyclique contenant de 1 à 10 atomes de carbone, éventuellement substitué par un ou des atomes d'halogène ou un ou des radicaux hydroxyle. On peut citer notamment les radicaux adamantyle ou 1-méthylcyclohéxyle.

Le radical thiocycloaliphatiqe préféré est le radical 1-adamantylthio.

Parmi les atomes d'halogène, on préfère le fluor ou le chlore.

L'étude des composés (I) dans plusieurs tests de mesure de l'activité neurotrophe a été effectuée.

### Effets des composés sur la production de NGF par des astrocytes en culture

Les cultures primaires d'astrocytes sont préparées à partir de cortex cérébral de rats nouveau-nés de 1 à 2 jours. Après dissection, les cortex sont dissociés mécaniquement par un passage au travers d'un nylon Blutex® (maille de 83 mM). Les cellules sont cultivées dans un milieu DMEM/F12 (Gibco, Brl 1:1) contenant 10 % de sérum de veau foetal, 2,5 mM de L-glutamine, 100 U/ml de penicilline et 100 mg/ml de streptomycine (1 cortex pour 30 ml) dans des boîtes Coming 12 puits à raison de 1 ml de préparation cellulaire par puits. Les boîtes sont placées à 37 °C dans un incubateur saturé à 5 % de CO₂, le milieu est remplacé tous les trois jours et les cultures sont utilisées le 7^{ème} jour de culture (80-90 % de confluence.). Les traitements avec les différents composés sont alors réalisés avec 500 ml par puits de milieu décrit ci-dessus pendant 40 h. A la fin du traitement le NGF, sécrété dans le milieu, est dosé par dosage immunoenzymatique et le taux des protéines membranaires est évalué par la technique de Bradford à l'aide du bleu de coomassie.

### Dosage du NGF

Les taux de NGF sont évalués par une technique immunoenzymatique. Des plaques de 96 puits sont traitées 2h à 37 °C par un anticorps monoclonal anti-NGF (0,67 mg/ml) solubilisé dans du tampon Na₂CO₃ 50 mM à pH 9,6. Les plaques vidées sont ensuite incubées 1 h à température de la pièce avec 1 % de sérum albumine bovine solubilisée dans le même tampon, et lavées avec un tampon Tris-HCI 50 mM (pH 7,0) contenant 200 mM de NaCI, 10 mM de CaCl₂, 0,1 % de Triton X-100 et 0,05 % de NaN₃ (Tampon A). Le milieu des astrocytes dilué au 9/10^{ème} dans un tampon d'extraction (50 mM Tris-HCI, pH 7, 200 mM NaCI, 1 % de BSA, 0,1 % de Triton X-100, 4 mg/ml aprotinin, 2 mM EDTA, 0,1 mM benzethonium chloride et 0,05 % NaN₃) et les standards de NGF (gamme de 10 à 320 pg/ml) sont alors ajoutés en duplicata (0,1 ml/puits). Les plaques sont incubées environ 16 h à 4 °C. Après un lavage avec le tampon A, les plaques sont incubées 4.h à 37 °C avec un deuxième anticorps monoclonal anti-NGF couplé à la β-galactosidase (0,13-0,15 U/ml). Après lavage avec le tampon A, les plaques sont incubées de 2 à 3 h à 37°C avec le substrat de la β-galactosidase, le chlorophénol rouge-β-D-galactopyranoside (2 mg/ml), préparé dans 10 mM d'HEPES (pH 7,0) contenant 150 mM NaCI, 2 mM MgCl₂, 0,1 % de NaN₃ et 1 % de BSA. Le produit de la réaction est mesuré à 570 nM par un lecteur de plaque Bio Kinetics Reader® EL 340.

### Résultats

Les composés de l'invention augmentent la production de NGF. A la concentration de 100 nM, les composés de l'invention les plus actifs augmentent la production de NGF jusqu'à plus de 70% par rapport aux témoins.

### Effets des composés sur la neuritogenèse et la croissance neuritique de ganglions de la racine dorsale

Les ganglions de la racine dorsale (DRG) sont prélevés sur des foetus de rat âgés de 14 jours. Les prélèvements sont effectués à 3 niveaux différents de la moelle (cervical, thoracique et sacré). Les DRG sont cultivés pendant 24 h dans un milieu DMEM (Gibco, Brl) contenant 5 % de sérum de veau foetal, 100 U/ml de pénicilline et 100 µg/ml de streptomycine, plus ou moins le composé à étudier dans un incubateur saturé à 5 % de CO₂. Le milieu est ensuite remplacé par un milieu identique dépourvu de sérum de veau foetal et contenant 1 µM de cytosine arabinoside (SIGMA) pour inhiber la prolifération des cellules non neuronales.

Les effets des composés sur la neuritogenése et la croissance neuritique sont évalués 48 h après la mise en culture par une technique morphométrique à l'aide d'un système d'analyse d'images BIOCOM.

### Résultats

Plusieurs composés de l'invention augmentent la neuritogenése d'explants de ganglions de la racine dorsale de plus de 100 % par rapport aux témoins, à la concentration de 100 nM.

Le potentiel thérapeutique des composés de l'invention pour le traitement des maladies auto-immunes du système nerveux a également été évalué.

### Effets des composés sur la prolifération des lymphocytes de souris présentant une encéphalite auto-immune expérimentale (EAE)

### Induction de l'EAE :

Des souris SJL/J femelles sont injectées en sous-cutané, à JO et J7 avec la protéine basique de la myéline (MBP, 400 µg/souris) émulsifiée dans de l'adjuvant complet de Freund. Environ 20 jours après la première immunisation, les ganglions lymphatiques des animaux qui développent les signes cliniques de l'EAE sont utilisés pour le test de prolifération.

### Mesure de la prolifération des lymphocytes :

Les ganglions sont prélevés et dissociés mécaniquement par un passage au travers d'un tamis métallique. Les cellules sont cultivées dans un milieu RPMI (Gibco, BRL) contenant 10 % de sérum de veau foetal décomplémenté, 2,5 mM de L-glutamine, 100 U/ml de pénicilline, 100 ug/ml de streptomycine et 0,5 % de 2-mercaptoéthanol à raison de 250 000 cellules par puits dans des boîtes 96 puits TPP. Les boîtes sont placées à 37°C dans un incubateur saturé à 5 % de CO₂. Les cellules sont traitées le jour de la culture avec les différents analogues de l'acide rétinoique en absence ou en présence de MBP (25 µg/ml ; stimulation antigénique) pendant 96 h. La prolifération est évaluée par la mesure de l'incorporation de thymidine tritiée ajoutée au milieu de culture durant les 16 dernières heures d'incubation.

### Résultats

L'analyse des résultats montre que les composés de l'invention inhibent la prolifération des lymphocytes provenant des souris EAE avec des Cl₅₀ allant de 0,1 à 100 nM.

### Effets des composés sur la production de TNF-α(Tumor Necrosis Factor-α) par les cellules microgliales activées.

### Cultures primaires de cellules microgliales :

Les cellules microgliales sont préparées à partir de cortex de rats nouveau-nés de 1 jour. Les cellules sont cultivées dans un milieu DMEM (Gibco, BRL) contenant 10 % de sérum de veau foetal (myoclone inactivé), 2,5 mM de L-glutamine, 4,5 g/l de L-glucose et 100 µg/ml de gentamycine dans des plaques 96 puits TPP. Les cellules sont traitées par les différents analogues de l'acide rétinoique et stimulées par 1 µg/ml de lipopolysaccharide (LPS) afin d'induire la production de TNFα. Après 18 h d'incubation les milieux de culture sont prélevés pour le dosage du TNFα.

### Dosage du TNFα :

Les taux de TNFα dans les surnageants de culture de cellules microgliales sont déterminés à l'aide d'un test de cytotoxicité sur la lignée cellulaire L929. Le test est révélé grâce au dosage colorimétrique du MTT.

### Résultats

Les composés de l'invention diminuent la production de TNFα par des cellules microgliales activées en culture, avec des effets allant jusqu'à 50 % de diminution à la concentration de 10nM.

Les résultats montrent que les composés de l'invention présentent une activité neurotrophe qui peut trouver une application pour le traitement des neuropathies périphériques de type traumatique, ischémique, métabolique, infectieux, alcoolique, iatrogénique ou génétique, et dans le traitement des maladies affectant les motoneurones, comme la sclérose latérale amyotrophique et les amyotrophies spinales. Ils peuvent aussi être utilisés pour la préparation de médicaments utiles dans le traitement des rétinopathies, de la sénilité cérébrale, de la démence consécutive aux infarctus multiples, de la démence vasculaire, dans le traitement de l'atrophie olivo-ponto-cérébelleuse et d'autres maladies neuro-dégénératives, par exemple la maladie d'Alzheimer, la maladie de Pick ou la chorée de Huntington. Les composés de l'invention peuvent aussi trouver une application dans la prévention de la mort neuronale faisant suite à un accident cérébro-vasculaire ou à un traumatisme médullaire ou crânien.

Ils peuvent également s'avérer actifs dans des pathologies d'origine auto-immune comme la sclérose en plaques, la maladie de Guillain-Barré et la myasténie gravis.

Les composés les plus actifs répondent à la formule suivante 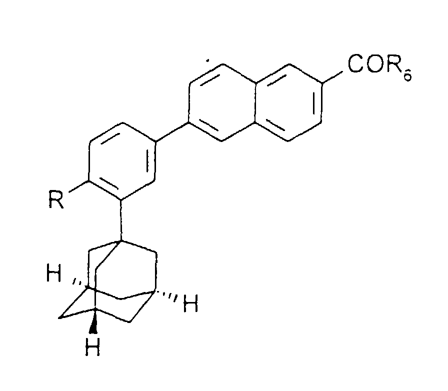 dans laquelle
- R₆ représente un radical 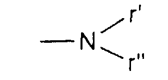 ou un radical OR₇, R₇ représentant un atome d'hydrogène, un radical alkyle ayant de 1 à 20 atomes de carbone, un radical monohydroxyalkyle, ou un radical polyhydroxyalkyle, r' et r" représentant un atome d'hydrogène, un radical alkyle inférieur, un radical mono ou polyhydroxyalkyle, un radical aryle éventuellement substitué ou un reste d'amino-acide ou de sucre aminé ou encore pris ensemble forment un héterocycle,
- R représente un atome d'hydrogène, un radical hydroxyle, un radical alkyle ramifié ou non ayant de 1 à 4 atomes de carbone ou un radical alcoxy de 1 à 4 atomes carbone.

Les acides 6-[3-(1-adamantyl)-4-méthoxyphényl]-2-naphtoïque, et 6-[3-(1-adamantyl)-4-hydroxyphényl]-2-naphtoïque, leurs esters (en particulier leurs esters méthyliques) et leurs amides sont les composés préférés.

Les composés peuvent être utilisés en association avec un ou plusieurs composés des classes suivantes : anti-inflammatoires stéroïdiens et non-stéroïdiens, anti-viraux (en particulier anti-herpès), immunosuppresseurs et immunomodulateurs.

Les composés peuvent être présentés sous toutes formes pharmaceutiques adaptées à l'administration entérale, parentérale ou locale, en association avec des excipients appropriés, par exemple sous forme de comprimés, dragées, gélules, capsules, suppositoires, patches, solutions ou suspensions buvables ou injectables. L'administration journalière peut varier de 0,01 à 100 mg de principe actif.

## Revendications

1. Utilisation des composés répondant à la formule (I) et de leurs sels, 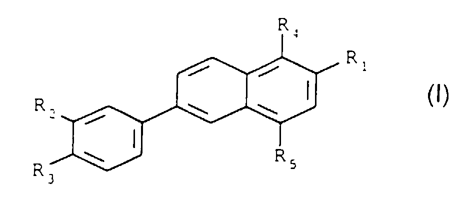 dans laquelle
- R₁ représente un groupe -COR₆ ou CH₂OH, R₆ représentant un radical 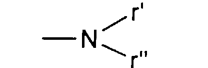 ou un radical OR₇, R₇ représentant un atome d'hydrogène, un radical alkyle ayant de 1 à 20 atomes de carbone, un radical monohydroxyalkyle, ou un radical polyhydroxyalkyle, r' et r" représentant un atome d'hydrogène, un radical alkyle inférieur, un radical mono ou polyhydroxyalkyle, un radical aryle éventuellement substitué ou un reste d'amino-acide ou de sucre aminé ou encore pris ensemble forment un hétérocycle,
- R₂ représente un atome d'hydrogène, un radical alkyle, ramifié ou non, ayant de 1 à 15 atomes de carbone, un radical alcoxy ayant de 1 à 4 atomes de carbone, ou un radical cycloaliphatique,
- R₃ représente un atome d'hydrogène, un radical hydroxyle, un radical alkyle, ramifié ou non, ayant de 1 à 4 atomes de carbone, un radical alcoxy ayant de 1 à 10 atomes de carbone,un radical cycloaliphatique substitué ou non, un radical thio-cycloaliphatique ou un radical de formule -O-Si(CH₃)₂-R₈, R₈ représentant un radical alkyle inférieur linéaire ou ramifié,
- R₄ et R₅, identiques ou différents, représentent un atome d'hydrogène,un radical alkyle inférieur, un radical hydroxyle ou un radical acyloxy inférieur, pour la fabrication de médicaments utiles dans le traitement des neuropathies périphériques, des maladies neurodégénératives centrales et des maladies auto-immunes du système nerveux.

2. Utilisation des composés répondant à la formule 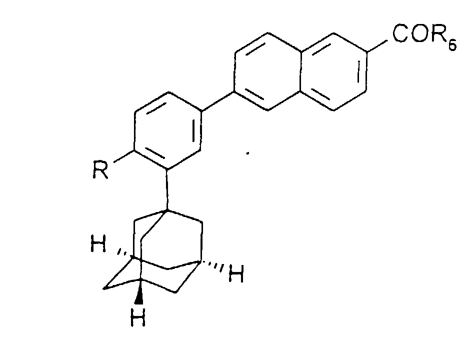 dans laquelle
- R₆ représente un radical 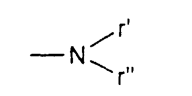 ou un radical OR₇, R₇ représentant un atome d'hydrogène, un radical alkyle ayant de 1 à 20 atomes de carbone, un radical monohydroxyalkyle, ou un radical polyhydroxyalkyle, r' et r" représentant un atome d'hydrogène, un radical alkyle inférieur, un radical mono ou polyhydroxyalkyle, un radical aryle éventuellement substitué ou un reste d'amino-acide ou de sucre aminé ou encore pris ensemble forment un héterocycle,
- R représente un atome d'hydrogène, un radical hydroxyle, un radical alkyle ramifié ou non ayant de 1 à 4 atomes de carbone ou un radical alcoxy de 1 à 4 atomes de carbone,
pour la fabrication de médicaments utiles dans le traitement des neuropathies périphériques, des maladies neurodégénératives centrales et des maladies auto-immunes du système nerveux.

3. Utilisation de l'acide 6-[3-(1-adamantyl)-4-méthoxyphényl]-2-naphtoïque, de ses esters et/ou de ses amides pour la fabrication de médicaments utiles dans le traitement des neuropathies périphériques, des maladies neurodégénératives centrales et des maladies auto-immunes du système nerveux.

4. Utilisation de l'acide 6-[3-(1-adamantyl)-4-hydroxyphényl]-2-naphtoïque, de ses esters et/ou de ses amides pour la fabrication de médicaments utiles dans le traitement des neuropathies périphériques, des maladies neurodégénératives centrales et des maladies auto-immunes du système nerveux.

## Claims

1. Use of the compounds corresponding to the formula I and of their salts, 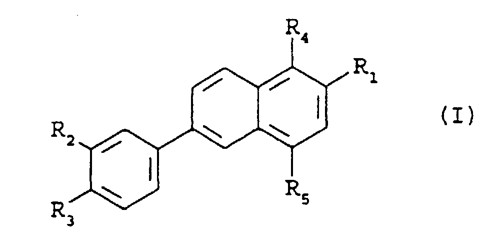 in which
- R₁ represents a group -COR₆ or CH₂OH, R₆ representing a radical 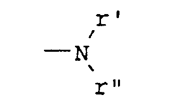 or a radical OR₇, R₇ representing a hydrogen atom, an alkyl radical having from 1 to 20 carbon atoms, a monohydroxyalkyl radical, or a polyhydroxyalkyl radical, r' and r" representing a hydrogen atom, a lower alkyl radical, a mono- or polyhydroxyalkyl radical, an optionally substituted aryl radical or an amino acid or amino sugar residue or alternatively taken together form a heterocycle,
- R₂ represents a hydrogen atom, a branched or unbranched alkyl radical having from 1 to 15 carbon atoms, an alkoxy radical having from 1 to 4 carbon atoms, or a cycloaliphatic radical,
- R₃ represents a hydrogen atom, a hydroxyl radical, a branched or unbranched alkyl radical having from 1 to 4 carbon atoms, an alkoxy radical having from 1 to 10 carbon atoms, a substituted or unsubstituted cycloaliphatic radical, a thiocycloaliphatic radical or a radical of formula -O-Si(CH₃)₂-R₈, R₈ representing a linear or branched lower alkyl radical,
- R₄ and R₅, which are identical or different, represent a hydrogen atom, a lower alkyl radical, a hydroxyl radical or a lower acyloxy radical, for the manufacture of medicaments for the treatment of peripheral neuropathies, central neurodegenerative diseases and autoimmune diseases of the nervous system.

2. Use of the compounds corresponding to the formula 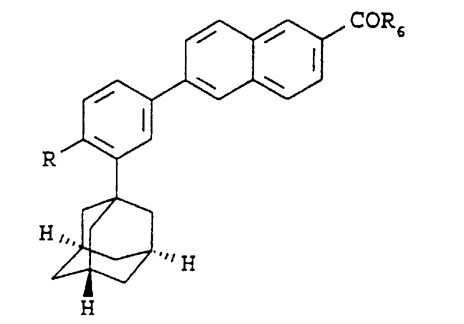 in which
- R₆ represents a radical 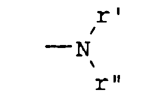 or a radical OR₇, R₇ representing a hydrogen atom, an alkyl radical having from 1 to 20 carbon atoms, a monohydroxyalkyl radical, or a polyhydroxyalkyl radical, r' and r" representing a hydrogen atom, a lower alkyl radical, a mono- or polyhydroxyalkyl radical, an optionally substituted aryl radical or an amino acid or amino sugar residue or taken together form a heterocycle,
- R represents a hydrogen atom, a hydroxyl radical, a branched or unbranched alkyl radical having from 1 to 4 carbon atoms or an alkoxy radical of 1 to 4 carbon atoms for the manufacture of medicaments for the treatment of peripheral neuropathies, central neurodegenerative diseases of the nervous system.

3. Use of 6-[3-(1-adamantyl)-4-methoxyphenyl]-2-naphthoic acid, its esters and/or its amides for the manufacture of medicaments for the treatment of peripheral neuropathies, central neurodegenerative diseases and autoimmune diseases of the nervous sytem.

4. Use of 6-[3-(1-adamantyl)-4-hydroxyphenyl]-2-naphthoic acid, its esters and/or its amides for the manufacture of medicaments for the treatment of peripheral neuropathies, central neurodegenerative diseases and autoimmune diseases of the nervous system.

## Patentansprüche

1. Verwendung von Verbindungen der Formel (I) und ihrer Salze zur Herstellung von Arzneimitteln, die zur Behandlung von peripheren Neuropathien, zentralen Neurodegenerationen und Autoimmunkrankheiten des Nervensystems geeignet sind: 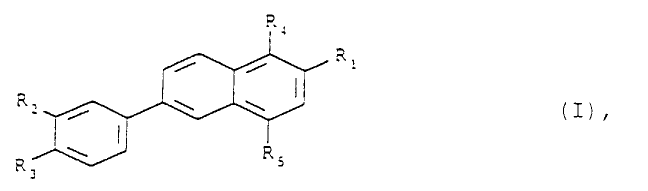 worin bedeuten:
- R₁ eine Gruppe -COR₆ oder CH₂OH, worin R₆ eine Gruppe 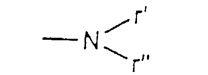 oder eine Gruppe OR₇ bedeutet, wobei R₇ ein Wasserstoffatom, eine Alkylgruppe mit 1 bis 20 Kohlenstoffatomen, eine Monohydroxyalkylgruppe oder eine Polyhydroxyalkylgruppe bedeutet und r' und r" ein Wasserstoffatom, eine niedere Alkylgruppe, eine Monooder Polyhydroxyalkylgruppe, eine gegebenenfalls substituierte Arylgruppe, einen Aminosäurerest oder einen Aminozuckerrest bedeuten oder gemeinsam einen Heterocyclus bilden;
- R₂ ein Wasserstoffatom, eine verzweigte oder nicht verzweigte Alkylgruppe mit 1 bis 15 Kohlenstoffatomen, eine Alkoxygruppe mit 1 bis 4 Kohlenstolfatomen oder eine cycloaliphatische Gruppe;
- R₃ ein Wasserstoffatom, eine Hydroxygruppe, eine verzweigte oder nicht verzweigte Alkylgruppe mit 1 bis 4 Kohlenstoffatomen, eine Alkoxygruppe mit 1 bis 10 Kohlenstoffatomen, eine gegebenenfalls substituierte cycloaliphatische Gruppe, eine thiocycloaliphatische Gruppe oder eine Gruppe der Formel -O-Si(CH₃)₂-R₈, wobei R₈ eine geradkettige oder verzweigte niedere Alkylgruppe bedeutet;
- R₄ und R₅, die identisch oder voneinander verschieden sind, ein Wasserstoffatom, eine niedere Alkylgruppe, eine Hydroxygruppe oder eine niedere Acyloxygruppe.

2. Verwendung von Verbindungen der Formel: 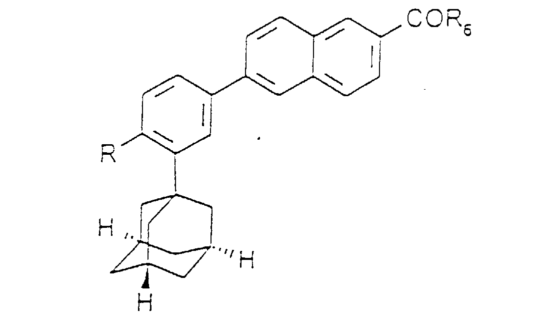 worin bedeuten:
- R₆ eine Gruppe 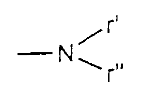 oder eine Gruppe OR₇, wobei R₇ ein Wasserstoffatom, eine Alkylgruppe mit 1 bis 20 Kohlenstoffatomen, eine Monohydroxyalkylgruppe oder eine Polyhydroxyalkylgruppe bedeutet und r' und r" ein Wasserstoffatom, eine niedere Alkylgruppe, eine Mono- oder Polyhydroxyalkylgruppe, eine gegebenenfalls substituierte Arylgruppe, einen Aminosäurerest oder einen Aminozukkerrest bedeuten oder gemeinsam einen Heterocyclus bilden;
- R ein Wasserstoffatom, eine Hydroxygruppe, eine verzweigte oder nicht verzweigte Alkylgruppe mit 1 bis 4 Kohlenstoffatomen oder eine Alkoxygruppe mit 1 bis 4 Kohlenstoffatomen,
zur Herstellung von Arzneimitteln, die zur Behandlung von peripheren Neuropathien, zentralen Neurodegenerationen und Autoimmunkrankheiten des Nervensystems geeignet sind.

3. Verwendung von 6-[3-(1-Adamantyl)-4-methoxyphenyl]-2-naphthoesäure, Estern und/oder Amiden dieser Verbindung zur Herstellung von Arzneimitteln, die zur Behandlung von peripheren Neuropathien, zentralen Neurodegenerationen und Autoimmunkrankheiten des Nervensystems geeignet sind.

4. Verwendung von 6-[3-(1-Adamantyl)-4-hydroxyphenyl]-2-naphthoesäure, Estern und/oder Amiden dieser Verbindung zur Herstellung von Arzneimitteln, die zur Behandlung von peripheren Neuropathien, zentralen Neurodegenerationen und Autoimmunkrankheiten des Nervensystems geeignet sind.
